# EUROPEAN PATENT APPLICATION

(11) **EP 4 775 597 A1**
(43) Date of publication of application: **15.07.2026**
(21) Application number: 24862918.0
(22) Date of filing: 06.09.2024
(51) Int. Cl.: C08B 15/00, A61K 9/20, A61K 45/00, A61K 47/38, C08J 3/12

(54) **CELLULOSE POWDER AND PHARMACEUTICAL COMPOSITION**

(30) Priority: 06.09.2023 JP 2023144760
(71) Applicant: ASAHI KASEI KABUSHIKI KAISHA, Tokyo 100-0006 (JP)
(72) Inventor: ITO, Yoshitaka, Tokyo 100-0006 (JP); HAYASHI, Yuji, Tokyo 100-0006 (JP); ITO, Takehiro, Tokyo 100-0006 (JP)
(74) Representative: Hoffmann Eitle
(86) International application number: PCT/JP2024/032087
(87) International publication number: WO 2025/053269

(57) **Abstract**

The present invention aims to provide a cellulose powder that can minimize an amount of nitrosamines generated when used as a raw material for pharmaceuticals, and a method for producing the same. The present invention also provides a cellulose powder having a nitrate ion content of 1.00 ppm or less and a sulfur content of 30.0 ppm or less, and a method for producing the cellulose powder by hydrolyzing, washing, and spray-drying a natural cellulosic material, wherein 1) the natural cellulosic material has a nitrite ion content of 0.010 ppm or less, a sulfur ion content of 30.0 ppm or less, and a nitrate ion content of 5.0 ppm or less, or 3) the spray-drying is carried out in a gas having a nitrogen dioxide content of 0.05 ppm or less or a nitrogen trioxide content of 0.05 ppm or less.

## Description

### TECHNICAL FIELD

The present invention relates to a cellulose powder for use in the production of pharmaceutical compositions having a reduced content of nitrosamines.

This application claims priority based on Japanese Patent Application No. 2023-144760, filed on September 6, 2023, the contents of which are incorporated herein by reference.

### BACKGROUND ART

Conventionally, in the fields of pharmaceuticals, foods, and other chemical industries, it has been widely practiced to prepare molded bodies containing active ingredients by using cellulose powders, such as crystalline cellulose and powdered cellulose, as excipients. These cellulose powders are required to have functionality that can improve various properties required for pharmaceuticals, such as good moldability and improved bioavailability of active ingredients.

For example, Patent Document 1 reports that by adjusting the powder properties of cellulose powder, in particular the average degree of polymerization, weight-average particle diameter, apparent specific volume, and the amount of organic carbon derived from residual impurities, defined as the total organic carbon amount (%) upon extraction with a 1% NaOH aqueous solution minus the total organic carbon amount (%) upon extraction with pure water, within specific ranges, it is possible to improve not only the compression moldability but also the flavor release of herbal medicines and the color development of sugar coating layers.

On the other hand, the carcinogenicity of nitrosamines has become a problem, and in recent years, there has been a strict requirement to control the content of nitrosamines in pharmaceuticals. Nitrite is the main component involved in the generation of nitrosamines in pharmaceuticals, and not only the bulk drug substance but also nitrite introduced by various additives such as excipients increases the risk of nitrosamine content exceeding the control index value (Non-Patent Document 1). For example, a synthetic pathway is known in which nitrosamines are generated by a chemical reaction between dimethylamine derived from a drug substance and nitrite derived from an additive at high temperatures.

### Citation List

### Patent Documents

Patent Document 1: Japanese Patent No. 6247207

### Non-Patent Documents

Non-Patent Document 1: Boetzel et al, Journal of Pharmaceutical Sciences, 2023, vol.112(6), p.1615-1624.

### SUMMARY OF INVENTION

### Technical Problem

The present invention provides a cellulose powder that has an extremely low content of components involved in the generation of nitrosamines and that can suppress the amount of nitrosamines generated when used as a raw material for pharmaceuticals; a method for producing the same; and a pharmaceutical composition made from the cellulose powder.

### Solution to Problem

The present inventors have conducted extensive research in light of the current situation described above, and have found that in addition to the conventionally known nitrite ion content, the content of nitrate ions, sulfur, hydrogen peroxide, and iron also affect the generation of nitrosamines. They have also found that by using a cellulose powder as a raw material in which the contents of these components have been adjusted to fall within appropriate ranges, the amount of nitrosamines generated during the manufacturing process and storage of pharmaceuticals can be kept lower than when other cellulose powders are used as a raw material, and have completed the present invention.

That is, the present invention includes the following aspects.
[1] A cellulose powder, which has a nitrate ion content of 1.00 ppm or less and a sulfur content of 30.0 ppm or less.
[2] The cellulose powder according to [1], which has a hydrogen peroxide content of 0.40 ppm or less.
[3] A cellulose powder, which has a nitrate ion content of 1.00 ppm or less and a hydrogen peroxide content of 0.40 ppm or less.
[4] The cellulose powder according to any one of [1] to [3], wherein the nitrite ion content is 0.200 ppm or less.
[5] The cellulose powder according to any one of [1] to [4], wherein an iron content is 0.10 ppm or more and 0.80 ppm or less.
[6] A pharmaceutical composition comprising the cellulose powder according to any one of [1] to [5] and a pharmaceutically active ingredient.
[7] The pharmaceutical composition according to [6], wherein the pharmaceutically active ingredient is a secondary amine, a tertiary amine, or a quaternary amine.
[8] The pharmaceutical composition according to [6] or [7], which is a tablet.
[9] A method for producing a cellulose powder, comprising
   hydrolyzing a natural cellulosic material, washing an insoluble residue in a cellulose dispersion after hydrolysis, and then spray-drying the washed cellulose, wherein
   (1) the natural cellulosic material has a nitrite ion content of 0.010 ppm or less or a sulfur ion content of 30.0 ppm or less, and a nitrate ion content of 5.0 ppm or less; or
   (3) the spray-drying is carried out in a gas having a nitrogen dioxide concentration of 0.05 ppm or less or a nitrogen trioxide concentration of 0.05 ppm or less.
[10] A method for producing a cellulose powder, comprising
   hydrolyzing a natural cellulosic material, washing an insoluble residue in a cellulose dispersion after hydrolysis, and then spray-drying the washed cellulose, wherein
   (1) the natural cellulosic material has a nitrite ion content of 0.010 ppm or less or a sulfur ion content of 30.0 ppm or less, and a nitrate ion content of 5.0 ppm or less; and
   (3) the spray-drying is carried out in a gas having a nitrogen dioxide concentration of 0.05 ppm or less.
[11] The method for producing a cellulose powder according to [9] or [10], wherein
   (2) water used in one or more processes selected from the group consisting of the hydrolysis reaction of the natural cellulosic material, the washing of the insoluble residue, and the spray-drying has a nitrite nitrogen content of 0.02 ppm or less, or a total content of nitrite nitrogen and nitrate nitrogen of 5.0 ppm or less.
[12] A method for producing a pharmaceutical composition, comprising using the cellulose powder according to any one of [1] to [5] and a pharmaceutically active ingredient as raw materials.
[13] The method for producing a pharmaceutical composition according to [12], wherein the cellulose powder is stored in a gas having a nitrogen dioxide concentration of 0.05 ppm or less and a nitrogen trioxide concentration of 0.05 ppm or less until a start of production.
[14] The method for producing a pharmaceutical composition according to [12] or [13], wherein the pharmaceutical composition is produced in a gas having a nitrogen dioxide concentration of 0.05 ppm or less and a nitrogen trioxide concentration of 0.05 ppm or less.
[15] The method for producing a pharmaceutical composition according to any one of [12] to [14], wherein the pharmaceutically active ingredient is a secondary amine, a tertiary amine, or a quaternary amine.
[16] The method for producing a pharmaceutical composition according to any one of [12] to [14], wherein the pharmaceutically active ingredient is a sartan compound, a compound having a dimethylaminomethyl group, or a biguanide compound.
[17] The method for producing a pharmaceutical composition according to any one of [12] to [16], wherein a mixture containing the cellulose powder and the pharmaceutically active ingredient is directly compressed into tablets, or granulated and then compressed into tablets.
[18] A method for producing a cellulose powder, comprising
   hydrolyzing a natural cellulosic material, washing an insoluble residue in a cellulose dispersion after hydrolysis, and then spray-drying the washed cellulose, wherein
   water used in one or more processes selected from the group consisting of the hydrolysis reaction of the natural cellulosic material, the washing of the insoluble residue, and the spray-drying has a nitrite nitrogen content of 0.02 ppm or less, or a total content of nitrite nitrogen and nitrate nitrogen of 5.0 ppm or less.

### Advantageous Effects of Invention

The cellulose powder of the above-mentioned embodiment has a low content of components that contribute to the generation of nitrosamines. Therefore, by using this cellulose powder as a raw material, it is possible to produce a pharmaceutical composition in which the amount of nitrosamines generated during the manufacturing process and storage is kept low.

### DESCRIPTION OF EMBODIMENTS

In the present invention and the present specification, unless otherwise specified, "ppm" means "mass ppm (0.001 mg/g)".

### <Cellulose powder>

The cellulose powder referred to in the present specification is generally referred to as crystalline cellulose, powdered cellulose, etc., and is suitable for use as a pharmaceutical additive or food additive. Among these, crystalline cellulose is preferable as the cellulose powder.

Known examples of the crystalline cellulose include microcrystalline cellulose described in the 9th edition of the Official Specification of Food Additives, crystalline cellulose described in the Japanese Pharmacopoeia (18th revision), and crystalline cellulose described in the United States Pharmacopoeia, the European Pharmacopoeia, etc.

The cellulose powder according to one embodiment of the present invention (hereinafter referred to as "the present embodiment") has a low content of components that contribute to the generation of nitrosamines, and when used as a raw material for products such as pharmaceuticals, it has a low ability to generate nitrosamines in the product (hereinafter sometimes referred to as "nitrosamine-generating ability"). Because the cellulose powder according to the present embodiment has a low nitrosamine-generating ability, it is suitable as a raw material for pharmaceuticals where the nitrosamine content is strictly limited.

The cellulose powder according to the present embodiment has a nitrate ion content (mass) of 1.00 ppm or less relative to the total amount (mass) of the cellulose powder. As shown in the Examples below, the nitrate ion content affects the amount of nitrosamines generated. The higher the nitrate ion content, the higher the amount of nitrosamines generated. However, by ensuring that the nitrate ion content (mass) of the cellulose powder is 1.00 ppm or less, preferably 0.50 ppm or less, and more preferably 0.20 ppm or less, the amount of nitrosamines generated when used as a pharmaceutical excipient can be kept low. The lower limit of the range of the nitrate ion content (mass) is 0.008 ppm, which is the detection limit. A nitrate ion content (mass) of less than 0.008 ppm in the cellulose powder is undetectable. The reason why the nitrate ion content affects the amount of nitrosamines generated is unclear, but it is presumed to be due to the reduction of nitrate ions in pharmaceuticals to nitrite ions during pharmaceutical manufacturing and storage after manufacturing.

The cellulose powder according to the present embodiment preferably has a lower nitrite ion content (mass). The nitrite ion content relative to the total amount (mass) of the cellulose powder according to the present embodiment is preferably 0.200 ppm or less, more preferably 0.100 ppm or less, even more preferably 0.050 ppm or less, even more preferably 0.020 ppm or less, and particularly preferably 0.015 ppm or less. The lower limit of the range of the nitrite ion content (mass) is 0.008 ppm, which is the detection limit. A nitrite ion content (mass) of less than 0.008 ppm in the cellulose powder is undetectable.

The cellulose powder according to the present embodiment has a sulfur content (mass) of 30.0 ppm or less relative to the total amount (mass) of the cellulose powder. As shown in the Examples below, the sulfur content affects the amount of nitrosamines generated. The higher the sulfur content, the higher the amount of nitrosamines generated. However, by ensuring that the sulfur content (mass) of the cellulose powder is 30.0 ppm or less, preferably 25.0 ppm or less, more preferably 20.0 ppm or less, and even more preferably 18.0 ppm or less, the amount of nitrosamines generated when used as a pharmaceutical excipient or the like can be kept low. While the reason why sulfur content affects the amount of nitrosamines generated is unclear, it is presumed that sulfur in pharmaceuticals functions as a reducing agent during pharmaceutical manufacturing and storage after manufacture, converting nitrate ions to nitrite ions. The lower limit of the sulfur content (mass) range is 0.10 ppm, which is the detection limit. A sulfur content (mass) of cellulose powder less than 0.10 ppm is undetectable.

The cellulose powder according to the present embodiment preferably has a hydrogen peroxide content (mass) of 0.40 ppm or less, more preferably 0.30 ppm or less, and even more preferably 0.20 ppm or less, relative to the total amount (mass) of the cellulose powder. The lower limit of the hydrogen peroxide content (mass) range is 0.10 ppm, which is the detection limit. A hydrogen peroxide content (mass) of less than 0.10 ppm in the cellulose powder is undetectable. As shown in the Examples below, the hydrogen peroxide content affects the amount of nitrosamines generated. The higher the hydrogen peroxide content, the higher the amount of nitrosamines generated. However, by keeping the hydrogen peroxide content (mass) relative to the total amount (mass) of the cellulose powder within the above range, the amount of nitrosamines generated can be kept low when the cellulose powder is used as a pharmaceutical excipient, etc. The reason why the hydrogen peroxide content affects the amount of nitrosamines generated is unclear; however, it is presumed that hydrogen peroxide in pharmaceuticals functions as a reducing agent, converting nitrate ions to nitrite ions during pharmaceutical manufacturing and storage after manufacture.

The cellulose powder according to the present embodiment preferably has an iron content (by mass) of 0.10 ppm to 0.80 ppm, more preferably 0.20 ppm to 0.80 ppm, even more preferably 0.30 ppm to 0.80 ppm, and even more preferably 0.20 ppm to 0.70 ppm, relative to the total amount (by mass) of the cellulose powder. In particular, the iron content (by mass) of the cellulose powder according to the present embodiment is more preferably 0.30 ppm to 0.70 ppm, even more preferably 0.30 ppm to 0.60 ppm. As shown in the Examples below, the iron content affects the amount of nitrosamines generated. While a low iron content increases the amount of nitrosamines generated, a high iron content is undesirable because it may increase the total amount of iron added. By keeping the iron content (by mass) relative to the total amount (by mass) of cellulose powder within the above range, it is possible to reduce the amount of nitrosamines generated when the cellulose powder is used as a pharmaceutical excipient, etc. The reason why the iron content affects the amount of nitrosamines generated is unclear, but it is presumed that this is because, during the pharmaceutical manufacturing process and storage after manufacture, iron in pharmaceuticals not only functions as a reducing agent but also reacts with nitrate ions to generate nitric oxide, thereby suppressing the conversion of nitrate ions to nitrite ions.

The contents of nitrate ions, nitrite ions, sulfur, hydrogen peroxide, and iron in the cellulose powder can all be measured by the methods described in the Examples below.

The cellulose powder according to the present embodiment is particularly preferably
a cellulose powder having a nitrate ion content of 1.00 ppm or less and a sulfur content of 30.0 ppm or less; a cellulose powder having a nitrate ion content of 1.00 ppm or less, a sulfur content of 30.0 ppm or less, and a hydrogen peroxide content of 0.40 ppm or less; a cellulose powder having a nitrate ion content of 1.00 ppm or less and a hydrogen peroxide content of 0.40 ppm or less;
a cellulose powder having a nitrate ion content of 1.00 ppm or less, a sulfur content of 30.0 ppm or less, and a nitrite ion content of 0.200 ppm or less; a cellulose powder having a nitrate ion content of 1.00 ppm or less, a sulfur content of 30.0 ppm or less, a hydrogen peroxide content of 0.40 ppm or less, and a nitrite ion content of 0.200 ppm or less; a cellulose powder having a nitrate ion content of 1.00 ppm or less, a hydrogen peroxide content of 0.40 ppm or less, and a nitrite ion content of 0.200 ppm or less;
a cellulose powder having a nitrate ion content of 1.00 ppm or less, a sulfur content of 30.0 ppm or less, and an iron content of 0.10 ppm or more and 0.80 ppm or less; a cellulose powder having a nitrate ion content of 1.00 ppm or less, a sulfur content of 30.0 ppm or less, a hydrogen peroxide content of 0.40 ppm or less, and an iron content of 0.10 ppm or more and 0.80 ppm or less; a cellulose powder having a nitrate ion content of 1.00 ppm or less, a hydrogen peroxide content of 0.40 ppm or less, and an iron content of 0.10 ppm or more and 0.80 ppm or less;
a cellulose powder having a nitrate ion content of 1.00 ppm or less, a sulfur content of 30.0 ppm or less, a nitrite ion content of 0.200 ppm or less, and an iron content of 0.10 ppm or more and 0.80 ppm or less; a cellulose powder having a nitrate ion content of 1.00 ppm or less, a sulfur content of 30.0 ppm or less, a hydrogen peroxide content of 0.40 ppm or less, a nitrite ion content of 0.200 ppm or less, and an iron content of 0.10 ppm or more and 0.80 ppm or less; or a cellulose powder having a nitrate ion content of 1.00 ppm or less, a hydrogen peroxide content of 0.40 ppm or less, a nitrite ion content of 0.200 ppm or less, and an iron content of 0.10 ppm or more and 0.80 ppm or less.

The average degree of polymerization of the cellulose powder according to the present embodiment is not particularly limited. The average degree of polymerization of the cellulose powder according to the present embodiment is preferably 100 or more and 350 or less, more preferably 150 or more and 300 or less, and even more preferably 180 or more and 250 or less. An average degree of polymerization of 100 or more is preferable because it improves moldability, and an average degree of polymerization of 350 or less is preferable because it does not exhibit fibrous properties and the powder has excellent fluidity and disintegration properties. In other words, an average degree of polymerization of 100 or more and 350 or less is preferable because it provides a particularly excellent balance between moldability, disintegration properties, and fluidity.

The weight-average particle diameter of the cellulose powder according to the present embodiment is not particularly limited. The weight-average particle diameter of the cellulose powder according to the present embodiment is preferably greater than 30 µm and less than 250 µm, more preferably greater than 30 µm and less than 180 µm, and even more preferably 40 µm or more and less than 150 µm. By setting the weight-average particle diameter to greater than 30 µm, preferably 40 µm or more, handling is improved without increasing adhesion and cohesion, and fluidity is also excellent. Furthermore, setting the weight-average particle diameter to 250 µm or less, preferably 180 µm or less, more preferably 150 µm or less, is preferable because it prevents separation and segregation from the active ingredient and does not deteriorate the content uniformity of the formulation.

The apparent specific volume of the cellulose powder according to the present embodiment is not particularly limited. The apparent specific volume of the cellulose powder according to the present embodiment is preferably 2 cm³/g or more and 15 cm³/g or less, more preferably 2 cm³/g or more and 13 cm³/g or less, even more preferably 2 cm³/g or more and 6 cm³/g or less, and particularly preferably 2 cm³/g or more and less than 4 cm³/g. An apparent specific volume of 2 cm³/g or more improves moldability. Since elastic recovery due to fibrous properties is exhibited, the upper limit is at most 15 cm³/g. In terms of improving fluidity and disintegrability, 6 cm³/g or less is preferable, and less than 4 cm³/g is even more preferable. The apparent specific volume of the cellulose powder according to the present embodiment is particularly preferably 2.3 cm³/g or more and 3.8 cm³/g or less, and even more preferably 3.0 cm³/g or more and 3.8 cm³/g or less.

The tapped apparent density of the cellulose powder according to the present embodiment is not particularly limited. The tapped apparent density of the cellulose powder according to the present embodiment is preferably 0.2 g/cm³ or more and 0.6 g/cm³ or less, more preferably 0.3 g/cm³ or more and 0.58 g/cm³ or less, and even more preferably 0.35 g/cm³ or more and 0.55 g/cm³ or less. A tapped apparent density of 0.6 g/cm³ or less improves moldability.

The angle of repose of the cellulose powder according to the present embodiment is not particularly limited. From the viewpoint of content uniformity, the angle of repose of the cellulose powder according to the present embodiment is preferably 36° or more and less than 44°, and more preferably 38° to 42°.

The physical properties of the cellulose powder can be measured as follows.
1) Average degree of polymerization (-)
   It can be measured by the copper ethylenediamine solution viscosity method described in the 18th edition of the Japanese Pharmacopoeia, Identification Test for Crystalline Cellulose (3).
2) Loss on drying (%)
   1 g of the powder is dried at 105°C for 3 hours, and the weight loss is expressed as a weight percentage.
3) Weight-average particle diameter of cellulose powder (µm)
   The weight-average particle diameter of a powder sample is measured by sieving 10 g of the sample for 10 minutes using a Rotap type sieve shaker (Sieve Shaker Type A manufactured by Hira Kosakusho Co., Ltd.) and a JIS standard sieve (Z8801-1987), and the particle size distribution is measured and expressed as the particle size at 50% of the cumulative weight.
4) Apparent specific volume (cm³/g)
   A 100 cm³ glass graduated cylinder was loosely filled with the powder sample over a period of 2 to 3 minutes using a quantitative feeder or the like, and the top surface of the powder layer was leveled horizontally with a soft brush, and the volume was recorded. This volume was divided by the weight of the powder sample to determine the value. The weight of the powder was appropriately determined so that the resulting volume falls within the range of approximately 70 to 100 cm³.
5) Apparent tapping density (g/cm³)
   Using a commercially available powder property measuring device (Hosokawa Micron, Powder Tester PT-R type), the powder was filled into a 100 cm³ cup, and after tapping 180 times, the volume of the cup was divided by the weight of the powder layer remaining in the cup to determine the powder property.
6) Angle of repose (°)
   Using a Sugihara-type angle of repose measuring device (slit size: depth 10 mm × width 50 mm × height 140 mm, protractor installed at the 50 mm width position), the dynamic free flow property was measured when cellulose powder was dropped into the slit using a quantitative feeder at a rate of 3 g/min. The angle of repose was the angle between the bottom of the device and the layer of cellulose powder formed.

### <Method of producing cellulose powder>

The cellulose powder according to the present embodiment can be produced, for example, by hydrolyzing a natural cellulosic material, washing the insoluble residue in the cellulose dispersion after hydrolysis, and then spray-drying. The reaction conditions for the hydrolysis reaction, the recovery and washing of the insoluble residue, and the spray-drying after re-dispersion can be determined by methods and conditions generally used for producing cellulose powders, or can be modified as appropriate.

The natural cellulosic material as the raw material may be of plant or animal origin. Examples of the natural cellulosic materials include cellulose-containing fibrous materials derived from natural sources such as wood, bamboo, wheat straw, rice straw, cotton, ramie, bagasse, kenaf, beet, sea squirt, bacterial cellulose, and the like. The natural cellulosic materials have a cellulose type I crystal structure. One of the above natural cellulosic materials may be used as the raw material, or a mixture of two or more may be used.

The natural cellulosic material used as the raw material is preferably used in the form of refined pulp, and from the viewpoint of production yield, refined pulp with an α-cellulose content of 85% or more is particularly preferred. There are no particular restrictions on the pulp refinement method, and any pulp, such as dissolving pulp, kraft pulp, or NBKP pulp, may be used. Wood-derived pulp is preferable from the viewpoints of high α-cellulose purity, ease of availability, and stable supply.

The hydrolysis of the natural cellulosic material may be performed by acid hydrolysis, alkaline oxidative decomposition, hydrothermal decomposition, or steam explosion. Any of these hydrolysis methods may be used alone, or two or more may be used in combination. Furthermore, the natural cellulosic material may be subjected to mechanical treatments such as pulverization, grinding, or the like, before or after hydrolysis.

Hydrolysis of natural cellulosic materials is carried out by dispersing solids containing the natural cellulosic material in a suitable medium. As the medium, water is preferable. The medium may be any medium other than water, as long as it is industrially used. For example, a mixture of water and an organic solvent may be used. Examples of such organic solvents include alcohols such as methanol, ethanol, isopropyl alcohol, butyl alcohol, 2-methylbutyl alcohol, benzyl alcohol, or the like; hydrocarbons such as pentane, hexane, heptane, cyclohexane, or the like; and ketones such as acetone, ethyl methyl ketone, or the like. As the organic solvents, those used in pharmaceuticals are particularly preferable, including those classified as solvents in the "Dictionary of Pharmaceutical Additives" (published by Yakuji Nipposha Co., Ltd.). Water and the organic solvents may be used alone or in combination of two or more. The material may be once dispersed in one medium, then the medium is removed and dispersed in a different medium.

When the hydrolysis of the natural cellulosic material is carried out by acid hydrolysis using hydrochloric acid, conditions such as hydrochloric acid concentration, hydrolysis temperature, and hydrolysis time are not particularly limited and are appropriately adjusted so as to obtain a cellulose powder with the desired physical properties. For example, the hydrochloric acid concentration is preferably 0.05 to 0.3%, more preferably 0.08 to 0.25%, and even more preferably 0.08 to 0.15%; the hydrolysis temperature is 100 to 150°C, preferably 80 to 150°C, and more preferably 100 to 150°C; and the hydrolysis time is preferably 40 to 150 minutes, and more preferably 70 to 110 minutes.

In general, when hydrolysis is performed, increasing the acid or alkali concentration of the hydrolysis solution or the reaction temperature tends to decrease the degree of cellulose polymerization and decrease the average particle diameter of cellulose in the dispersion. Furthermore, increasing the stirring force of the solution also tends to decrease the average particle diameter of cellulose particles in the dispersion. Therefore, by adjusting the degree of polymerization of the raw cellulose and the stirring force during the hydrolysis or dispersion process of the natural cellulosic material, the degree of polymerization and average particle diameter of the cellulose particles can be controlled within a desired range. The stirring force depends on the width, height, and volume of the stirring bed, the type and diameter of the blades, the stirring rotation speed, etc.

The cellulose dispersion obtained after hydrolysis is subjected to solid-liquid separation to recover insoluble residue, which is then washed. At this time, neutralization may be performed by alkali treatment or acid treatment, if necessary. The washing liquid used in the washing treatment may be the same medium as that used to disperse the natural cellulosic material. Furthermore, when washing treatment is performed multiple times, different washing liquids may be used for each washing. In the production of the cellulose powder according to the present embodiment, washing with pure water is preferable.

The insoluble residue after washing is dispersed again in pure water to prepare a cellulose dispersion. The cellulose dispersion is spray-dried to produce a cellulose powder. The redispersed cellulose dispersion may be subjected to mechanical treatments such as pulverization or grinding, centrifugation using a cyclone or centrifuge, or separation using a sieve before spray-drying. These treatment methods may be used alone or in combination of two or more.

Examples of the pulverization method include screen pulverization methods such as a screen mill and a hammer mill; blade rotary shear screen pulverization methods such as a flash mill; airflow pulverization methods such as a jet mill; ball pulverization methods such as a ball mill and a vibration ball mill; blade stirring pulverization methods; and the like.

Examples of the grinding method include grinding methods using stirring blades such as unidirectional rotation, multi-axis rotation, reciprocating inversion, up-down movement, rotation and up-down movement, and pipeline type blades, such as a portable mixer, a three-dimensional mixer, a side mixer, etc.; jet-type stirring and grinding methods such as a line mixer, etc.; grinding methods using a high-shear homogenizer, a highpressure homogenizer, an ultrasonic homogenizer, etc.; and shaft rotation extrusion grinding methods such as a kneader; and the like.

As the method for spray-drying the redispersed cellulose dispersion, various methods can be used, such as a disk type, a pressurized nozzle, a pressurized two-fluid nozzle, a pressurized four-fluid nozzle, or the like. These spray methods can be used alone or in combination of two or more. The spray-drying temperature may be a commonly used inlet temperature of 150°C to 300°C. Furthermore, freeze drying, drum drying, shelf drying, flash drying, vacuum drying, etc. can also be used instead of or in combination with spray-drying.

When carrying out the above-mentioned spray-drying, a small amount of a water-soluble polymer or a surfactant may be added to the dispersion in order to reduce the surface tension of the dispersion, and a foaming agent or gas may be added to the dispersion in order to accelerate the evaporation rate of the medium.

Examples of the water-soluble polymers include those listed in the "Dictionary of Pharmaceutical Additives " (published by Yakuji Nipposha Co., Ltd.), such as hydroxypropyl cellulose, hydroxypropylmethyl cellulose, polyacrylic acid, carboxyvinyl polymer, polyethylene glycol, polyvinyl alcohol, polyvinylpyrrolidone, methyl cellulose, gum arabic, starch paste, or the like. These water-soluble polymers may be used alone or in combination of two or more.

Examples of the surfactants include those classified as surfactants in the "Dictionary of Pharmaceutical Additives" (published by Yakuji Nipposha Co., Ltd.), such as a phospholipid, a glycerin fatty acid ester, a polyethylene glycol fatty acid ester, a sorbitan fatty acid ester, a polyoxyethylene hydrogenated castor oil, a polyoxyethylene cetyl ether, a polyoxyethylene stearyl ether, a polyoxyethylene nonylphenyl ether, a polyoxyethylene polyoxypropylene glycol, a polyoxyethylene sorbitan monolaurate, a polysorbate, a sorbitan monooleate, a glyceride monostearate, a monooxyethylene sorbitan monopalmitate, a monooxyethylene sorbitan monostearate, a polyoxyethylene sorbitan monooleate, a sorbitan monopalmitate, a sodium lauryl sulfate, or the like. These surfactants may be used alone or in combination of two or more.

Examples of the foaming agents include those listed in the "Dictionary of Pharmaceutical Additives " (published by Yakuji Nipposha Co., Ltd.), such as tartaric acid, sodium bicarbonate, potato starch, anhydrous citric acid, medicated soap, sodium lauryl sulfate, lauric acid diethanolamide, lauromacrogol, or the like. These foaming agents may be used alone or in combination of two or more.

In addition to the pharmaceutical additives, other compounds may also be used, such as bicarbonates that generate gas upon thermal decomposition, such as sodium bicarbonate, ammonium bicarbonate, or the like; carbonates such as sodium carbonate, ammonium carbonate, or the like that generate gas upon reaction with an acid; or the like. However, when using the above carbonates, they must be used together with an acid. Examples of the acids include organic acids such as citric acid, acetic acid, ascorbic acid, adipic acid, or the like; protonic acids such as hydrochloric acid, sulfuric acid, phosphoric acid, nitric acid, or the like; Lewis acids such as boron fluoride, or the like; and the like. Of these, acids used in pharmaceuticals or foods are preferable, but other acids also have similar effects.

Alternatively, instead of the foaming agent, a gas such as nitrogen, carbon dioxide, liquefied petroleum gas, dimethyl ether, or the like may be impregnated into the dispersion.

The water-soluble polymer, surfactant, and gas-generating substance such as the foaming agent may be added before drying, and there is no particular limitation on the timing of their addition.

In the method for producing a cellulose powder according to the present embodiment, at least one of the following (1) and (3) is carried out in order to keep the content of components that contribute to the production of nitrosamines low and produce cellulose powder with low nitrosamine-generating ability.
(1) The natural cellulosic material has a nitrite ion content of 0.010 ppm or less or a sulfur ion content of 30.0 ppm or less, and a nitrate ion content of 5.0 ppm or less.
(3) The spray-drying is carried out in a gas having a nitrogen dioxide concentration of 0.05 ppm or less or a nitrogen trioxide concentration of 0.05 ppm or less.

In the method for producing a cellulose powder according to the present embodiment, it is also preferable to carry out the following (2) in addition to at least one of the above (1) and (3).

(2) The water used in one or more processes selected from the group consisting of the hydrolysis reaction of the natural cellulosic material, the washing of the insoluble residue, and the spray-drying has a nitrite nitrogen content of 0.02 ppm or less, or a total content of nitrite nitrogen and nitrate nitrogen of 5.0 ppm or less.

The majority of nitrite ions and nitrate ions in cellulose powder originate from the natural cellulosic material used as the raw material. Therefore, in the present embodiment, a cellulose powder with reduced nitrosamine-generating ability can be produced by using a natural cellulosic material with a low content of nitrite ions, sulfur ions, or nitrate ions as the raw material. In the present embodiment, it is preferable to use a natural cellulosic material with a nitrite ion content of 0.010 ppm or less and a nitrate ion content of 5.0 ppm or less as the raw material, or a natural cellulosic material with a sulfur ion content of 30.0 ppm or less and a nitrate ion content of 5.0 ppm or less as the raw material.

The method for measuring trace substances such as nitrite ions, sulfur ions, and nitrate ions contained in the raw natural cellulosic material is not particularly limited, and any known method may be used. For example, the measurement can be performed using the same method as the measurement of trace substances in the cellulose powder described above.

In the measuring of trace substances in the natural cellulosic materials, it is preferable to perform pretreatment such as treating the pulp to a flocculated state in order to improve extraction efficiency and evaluation accuracy. There are no limitations on the method for treating the pulp to a flocculated state, and it can be treated using a general grinder. For example, a high-speed mill manufactured by LabNext or a home mixer may be used.

The amounts of nitrite ions, sulfur ions, and nitrate ions in the cellulose powder are affected by the water used in the reaction solution for the hydrolysis reaction, the water used in the washing solution for washing the insoluble residue, and the water used to prepare the cellulose dispersion to be subjected to spray-drying. In the present embodiment, by using water with a low content of nitrite ions and nitrate ions, the amount introduced from the water can be kept low, making it possible to produce a cellulose powder with an extremely low content of nitrite ions and nitrate ions and low nitrosamine-generating ability. More specifically, it is preferable to use water with a nitrite nitrogen content of 0.02 ppm or less, or water with a total content of nitrite nitrogen and nitrate nitrogen of 5.0 ppm or less.

In the method for producing a cellulose powder according to the present embodiment, it is preferable that the water used in any one of the hydrolysis reaction of the natural cellulosic material, washing of the insoluble residue, and spray-drying be water with a low content of nitrite ions, etc. It is more preferable that the water used in any two of the hydrolysis reaction of the natural cellulosic material, washing of the insoluble residue, and spray-drying be water with a low content of nitrite ions, etc. It is particularly preferable that the water used in all of the hydrolysis reaction of the natural cellulosic material, washing of the insoluble residue, and spray-drying be water with a low content of nitrite ions, etc.

The amount of nitrite ions and nitrate ions in the cellulose powder is affected by the gas used during the spray-drying. In the present embodiment, the spray-drying is performed in a gas containing almost no nitrite ions or nitrate ions, thereby minimizing the amount of nitrite ions and nitrate ions introduced from the gas. This allows the production of a cellulose powder with a very low content of nitrite ions and nitrate ions and low nitrosamine-generating ability. In the method for producing a cellulose powder according to the present embodiment, the NOx (nitrogen oxide) concentration of the gas during the spray-drying is preferably 0.00 ppm or more and 0.20 ppm or less, more preferably 0.00 ppm or more and 0.15 ppm or less, and even more preferably 0.00 ppm or more and 0.10 ppm or less. The NOx concentration of the gas is primarily the total concentration of nitric oxide, nitrogen dioxide, nitrous oxide, and dinitrogen trioxide.

In the method for producing a cellulose powder according to the present embodiment, it is preferable to use, as the gas used during the spray-drying, "a gas having a nitrogen dioxide concentration of 0.05 ppm or less" or "a gas having a nitrogen trioxide concentration of 0.05 ppm or less", and it is particularly preferable to use "a gas having a nitrogen dioxide concentration of 0.05 ppm or less and a nitrogen trioxide concentration of 0.05 ppm or less". As the "gas having a nitrogen dioxide concentration of 0.05 ppm or less and a nitrogen trioxide concentration of 0.05 ppm or less", for example, an atmosphere in which the nitrogen dioxide concentration is controlled to 0.05 ppm or less and the nitrogen trioxide concentration is controlled to 0.05 ppm or less is used.

The nitrogen dioxide concentration of the gas during the spray-drying is preferably from 0.000 ppm to 0.050 ppm, more preferably from 0.00 ppm to 0.025 ppm, and even more preferably from 0.00 ppm to 0.020 ppm. The nitrogen trioxide concentration of the gas during the spray-drying is preferably from 0.000 ppm to 0.050 ppm, more preferably from 0.00 ppm to 0.025 ppm, and even more preferably from 0.00 ppm to 0.020 ppm.

The method for controlling the nitrogen dioxide and nitrogen trioxide concentrations in the atmosphere is not particularly limited as long as it is a method capable of removing nitrogen dioxide and nitrogen trioxide from the atmosphere. Examples of the methods for removing nitrogen dioxide and nitrogen trioxide from dry air include a wet adsorption method using a scrubber and a dry adsorption method using a chemical filter, and the like.

In the method using a chemical filter, it is important to select an adsorbent that can efficiently remove acidic gases such as nitrogen dioxide. Examples of the adsorbents that can efficiently remove acidic gases include "SAAF Carb" (manufactured by AAF), "Pure Smel Filter Adsorbent E3" and "Pure Smel Filter Adsorbent E5" (both manufactured by Nippon Mokukai Co., Ltd.), "Gigacol" and "CP Blend Select" (both manufactured by Nitta Corporation), "Phylofresh VZG" and "Phylofresh VCL" (both manufactured by Nippon Vilene Co., Ltd.), and "RM2B90" (manufactured by Osaka Gas Chemicals Co., Ltd.). An appropriate adsorbent can be selected depending on the size and production capacity of the cellulose powder production equipment. There are no limitations on the method for selecting an adsorbent, as long as it can adjust the amount of nitrogen dioxide contained in dry air to a certain value or less.

In the method for producing a cellulose powder according to the present embodiment, it is preferable that not only the gas used for the spray-drying but also the steps other than the spray-drying in the cellulose powder production process be carried out in a gas in which the nitrogen dioxide concentration is controlled to 0.05 ppm or less or the nitrogen trioxide concentration is controlled to 0.05 ppm or less, and it is more preferable that all steps for producing a cellulose powder be carried out in a gas in which the nitrogen dioxide concentration is controlled to 0.05 ppm or less or the nitrogen trioxide concentration is controlled to 0.05 ppm or less. By producing in air in which the concentrations of nitrogen dioxide and nitrogen trioxide are controlled to be low, the introduction of nitrite nitrogen and nitrate nitrogen from the air is suppressed, and a cellulose powder with a low content of nitrite ions and nitrate ions can be stably produced.

The method for measuring the concentrations of nitrogen dioxide and nitrogen trioxide in the air is not particularly limited, and measurements can be made by conventional methods. For example, the concentrations of nitrogen monoxide, nitrogen dioxide, and NOx in the air can be measured using a general NOx measuring device that employs a chemiluminescence method. Examples of the NOx measuring devices include the "GLN-354D" (manufactured by DKK-TOA Corporation), the "APNA-370" (manufactured by Horiba, Ltd.), the "NOA-308Dx" (manufactured by Shimadzu Corporation), and the like. In addition, the concentrations of nitrogen dioxide and nitrogen trioxide in the air can be measured by measuring samples collected using a diffusion sampler for collecting acidic gases using ion chromatography or the Satzmann method.

In the method for producing a cellulose powder according to the present embodiment, at least one of the above-mentioned (1) and (3) may be carried out. In the method for producing a cellulose powder according to the present embodiment, it is preferable to carry out the above-mentioned (1) and (3), since this allows the content of components that contribute to the production of nitrosamines to be kept sufficiently low.

In the method for producing a cellulose powder according to the present embodiment, it is also preferable to carry out (2) in addition to at least one of (1) and (3).

Even when a cellulose powder is produced by carrying out only the above (2), it is possible to produce a cellulose powder with a reduced content of components that contribute to the production of nitrosamines.

Specifically, by using a natural cellulosic material as the raw material that satisfies the above (1), it is possible to produce a cellulose powder with an extremely low content of components that contribute to the generation of nitrosamines.

By carrying out the spray-drying in a gas that satisfies the above (3), it is also possible to produce a cellulose powder with an extremely low content of components that contribute to the generation of nitrosamines.

By using water whose nitrite nitrogen content or the total content of nitrite nitrogen and nitrate nitrogen satisfies the above-mentioned (2) as the water used in one or more processes selected from the group consisting of the hydrolysis reaction of the natural cellulosic materials, the washing of the insoluble residues, and the spray-drying, it is possible to produce a cellulose powder with an extremely low content of components that contribute to the generation of nitrosamines.

In order to more sufficiently reduce the content of components that contribute to the production of nitrosamines, it is preferable to carry out at least two of the steps (1) to (3) in the method for producing a cellulose powder according to the present embodiment, and it is particularly preferable to carry out all of the steps (1) to (3).

In the method for producing a cellulose powder according to the present embodiment, in addition to at least one of the above (1) to (3), it is also preferable to carry out various treatments to reduce the amounts of sulfur, hydrogen peroxide, and iron introduced into the final cellulose powder. For example, as the water used for washing the insoluble residues and during the subsequent spray-drying, water with a low content of not only nitrite and nitrate nitrogen but also sulfur, hydrogen peroxide, and iron (e.g., pure water) may be used. Furthermore, the number of washes for the insoluble residues may be set to be higher than conventional levels, for example, 3 times or more, preferably 4 times or more, more preferably 5 times or more, and even more preferably 6 times or more. The more washes, the more the amounts of sulfur, hydrogen peroxide, and iron introduced from the raw materials can be reduced.

The cellulose powder according to the present embodiment, like other cellulose powders, can be used as a raw material for various products such as pharmaceuticals, foods and beverages, feed, cosmetics, sanitary products, agricultural chemicals, etc. The cellulose powder according to the present embodiment has a low ability to generate nitrosamines, which have a significant impact on human health, and is therefore particularly suitable as an excipient for pharmaceuticals and foods and beverages whose nitrosamine content is limited.

### <Pharmaceutical composition>

The pharmaceutical composition according to the present embodiment contains the cellulose powder according to the present embodiment and a pharmaceutically active ingredient. The contents of the pharmaceutically active ingredient and the cellulose powder according to the present embodiment in the pharmaceutical composition are not particularly limited, and in typical use ranges, the content of the pharmaceutically active ingredient is 0.001% by mass or more and 99.0% by mass or less, and the content of the cellulose powder according to the present embodiment is 1.0% by mass or more and 99.0% by mass or less, relative to the total mass of the pharmaceutical composition. By ensuring that the content of the pharmaceutically active ingredient is equal to or greater than the above-mentioned lower limit, a therapeutically effective amount can be ensured. On the other hand, by ensuring that the content is equal to or less than the above-mentioned upper limit, the content of the cellulose powder according to the present embodiment can be equal to or greater than the above-mentioned lower limit, thereby making it possible to take full advantage of the feature of the cellulose powder according to the present embodiment that it is less likely to generate nitrosamines.

The pharmaceutically active ingredient contained in the pharmaceutical composition according to the present embodiment is not particularly limited. Furthermore, the pharmaceutical composition may contain only one type of pharmaceutically active ingredient, or two or more types. Examples of the pharmaceutically active ingredient include orally administered drugs such as hyperlipidemic drugs, diabetes drugs, stomachics, antacids, digestive drugs, antipyretic analgesics, anti-inflammatory drugs, hypnotics, sedatives, antisomnia drugs, antivertigo drugs, pediatric analgesics, cardiac stimulants, antiarrhythmic drugs, antihypertensive drugs, vasodilators, diuretics, antiulcer drugs, intestinal regulators, osteoporosis drugs, antitussives and expectorants, antiasthmatic drugs, antibacterial agents, agents for improving frequent urination, tonics, vitamins, or the like.

The pharmaceutically active ingredient contained in the pharmaceutical composition according to the present embodiment is preferably a secondary amine, a tertiary amine, or quaternary amine that is prone to generating nitrosamines. In particular, pharmaceutically active ingredients for which the content of nitrosamines, such as NDMA (N-nitrosodimethylamine) and NDEA (N-nitrosodiethylamine), is considered problematic are preferable. Examples of such pharmaceutically active ingredients include sartan compounds (e.g., valsartan, irbesartan, olmesartan, losartan, etc.), compounds containing a dimethylaminomethyl group (e.g., ranitidine, nizatidine, d-chlorpheniramine maleate (MCPA)), etc.), and biguanide compounds (e.g., metformin, buformin, etc.).

Among the secondary amine, tertiary amine, and quaternary amine, the pharmaceutically active ingredient contained in the pharmaceutical composition according to the present embodiment is preferably at least one selected from the group consisting of
sitagliptin, rifampicin, gliclazide, sitagliptin, orphenadrine, alpraziquantel, ropivacaine, ambroxol, quetiapine, atomoxetine, atenolol, azithromycin, betahistine, benazepril, bisoprolol, bumetanide, bupropion, cilazapril, ciprofloxacin, dabigatran, desloratadine, trimebutine, azithromycin, chloropyramine,
triprolidine, diclofenac, duloxetine, enalapril, fluoxetine, hydrochlorothiazide, ketamine, labetalol, landiolol, levofloxacin, lisinopril, mefenamic acid, methylphenidate, metoprolol, mirabegron, moxifloxacin, nebivolol, valacyclovir,
amitriptyline, nortriptyline, paroxetine, perindopril, phenylephrine, pracipexole, propranolol, pseudoephedrine, quinapril, ramipril, rasagiline, reboxetine, salbutamol, sertraline, sotalol, tamsulosin, trimetazidine, varenicline, vildagliptin, and vortioxetine; and more preferably at least one selected from the group consisting of
sitagliptin, rifampicin, orphenadrine, alpraziquantel, ropivacaine, ambroxol, quetiapine, atenolol, betahistine, benazepril, bisoprolol, bumetanide, bupropion, cilazapril, ciprofloxacin, dabigatran, desloratadine, trimebutine, chloropyramine,
triprolidine, diclofenac, enalapril, ketamine, labetalol, landiolol, levofloxacin, lisinopril, metoprolol, mirabegron, moxifloxacin, nebivolol, valacyclovir,
perindopril, phenylephrine, pracipexole, propranolol, pseudoephedrine, ramipril, rasagiline, salbutamol, sertraline, sotalol, tamsulosin, trimetazidine, vildagliptin, and vortioxetine.

The form of the pharmaceutical composition according to the present embodiment is not particularly limited and may be any of tablets, powders, fine granules, granules, extracts, pills, etc. Among these, tablets and granules are preferable because a large amount of cellulose powder is used per product. For example, a tablet containing the pharmaceutically active ingredient and the cellulose powder according to the present embodiment can be obtained by processing the pharmaceutically active ingredient and the cellulose powder according to the present embodiment by a known method such as mixing, stirring, granulating, sizing, and tableting.

The pharmaceutical composition according to the present embodiment may contain, in addition to the pharmaceutically active ingredient and the cellulose powder according to the present embodiment, excipients, disintegrants, binders, flow agents, lubricants, flavoring agents, fragrances, colorants, and sweeteners, as needed.

Examples of the excipients include those classified as excipients in the "Dictionary of Pharmaceutical Additives" (published by Yakuji Nipposha Co., Ltd.), such as starch acrylate, L-aspartic acid, aminoethylsulfonic acid, aminoacetic acid, powdered starch syrup, gum arabic, gum arabic powder, alginic acid, sodium alginate, pregelatinized starch, pumice grains, inositol, ethyl cellulose, ethylene vinyl acetate copolymer, sodium chloride, olive oil, kaolin, cocoa butter, casein, fructose, pumice grains, carmellose, carmellose sodium, hydrated silicon dioxide, dried yeast, dried aluminum hydroxide gel, dried sodium sulfate, dried magnesium sulfate, agar, powdered agar, xylitol, citric acid, sodium citrate, disodium citrate, glycerin, calcium glycerophosphate, sodium gluconate, L-Glutamine, clay, clay 3, clay granules, croscarmellose sodium, crospovidone, magnesium aluminosilicate, calcium silicate, magnesium silicate, light anhydrous silicic acid, light liquid paraffin, cinnamon powder, microcrystalline cellulose, microcrystalline cellulose/carmellose sodium, microcrystalline cellulose (granules), brown rice koji, synthetic aluminum silicate, synthetic hydrotalcite, sesame oil, wheat flour, wheat starch, wheat germ flour, rice flour, rice starch, potassium acetate, calcium acetate, cellulose acetate phthalate, safflower oil, white beeswax, zinc oxide, titanium oxide, magnesium oxide, β-cyclodextrin, dihydroxyaluminum aminoacetate, 2,6-dibutyl-4-methylphenol, dimethylpolysiloxane, tartaric acid, potassium hydrogen tartrate, calcined gypsum, sucrose fatty acid ester, magnesium alumina hydroxide, aluminum hydroxide gel, aluminum hydroxide/sodium bicarbonate coprecipitate, magnesium hydroxide, squalane, stearyl alcohol, stearic acid, calcium stearate, polyoxyethylene stearate, magnesium stearate, hardened soybean oil, refined gelatin, refined shellac, refined white sugar, refined white sugar spheres, cetostearyl alcohol, polyethylene glycol 1000 monocetyl ether, gelatin, sorbitan fatty acid ester, D-sorbitol, tricalcium phosphate, soybean oil, soybean unsaponifiables, soybean lecithin, skim milk powder, talc, ammonium carbonate, calcium carbonate, magnesium carbonate, neutral anhydrous sodium sulfate, low-substituted hydroxypropyl cellulose, dextran, dextrin, natural aluminum silicate, corn starch, tragacanth powder, silicon dioxide, calcium lactate, lactose, lactose granules, perfiller 101, white shellac, white petrolatum, whit clay, white sugar, white sugar and starch spherical granules, naked barley leaf extract powder, dried naked malt leaf green juice powder, honey, paraffin, potato starch, semi-digested starch, human serum albumin, hydroxypropyl starch, hydroxypropyl cellulose, hydroxypropyl cellulose, hydroxypropyl methylcellulose phthalate, hydroxypropyl methylcellulose phthalate, phytic acid, glucose, glucose hydrate, partially pregelatinized starch, pullulan, propylene glycol, powdered reduced maltose syrup, powdered cellulose, pectin, bentonite, sodium polyacrylate, polyoxyethylene alkyl ether, polyoxyethylene hydrogenated castor oil, polyoxyethylene (105) polyoxypropylene (5) glycol, polyoxyethylene (160) polyoxypropylene (30) glycol, sodium polystyrene sulfonate, polysorbate 80, polyvinyl acetal diethylaminoacetate, polyvinylpyrrolidone, polyethylene glycol, maltitol, maltose, D-mannitol, starch syrup, isopropyl myristate, anhydrous lactose, anhydrous calcium hydrogen phosphate, anhydrous calcium phosphate granules, magnesium aluminometasilicate, methylcellulose, cottonseed flour, cottonseed oil, Japan wax, aluminum monostearate, glycerin monostearate, sorbitan monostearate, medicinal charcoal, peanut oil, aluminum sulfate, calcium sulfate, granular corn starch, liquid paraffin, dl-malic acid, calcium hydrogen phosphate, calcium hydrogen phosphate, calcium hydrogen phosphate granules, sodium hydrogen phosphate, potassium dihydrogen phosphate, or calcium dihydrogen phosphate. These excipients may be used alone or in combination of two or more.

Examples of the disintegrants include those classified as disintegrants in the "Dictionary of Pharmaceutical Additives" (published by Yakuji Nipposha Co., Ltd.), such as celluloses such as croscarmellose sodium, carmellose, carmellose calcium, carmellose sodium, and low-substituted hydroxypropyl cellulose; starches such as carboxymethyl starch sodium, hydroxypropyl starch, rice starch, wheat starch, corn starch, potato starch, and partially pregelatinized starch; and synthetic polymers such as crospovidone and crospovidone copolymer. These disintegrants may be used alone or in combination of two or more.

Examples of the binders include those classified as binders in the "Dictionary of Pharmaceutical Additives" (published by Yakuji Nipposha Co., Ltd.), such as sugars such as sucrose, glucose, lactose, fructose, or the like; sugar alcohols such as mannitol, xylitol, maltitol, erythritol, sorbitol, or the like; water-soluble polysaccharides such as gelatin, pullulan, carrageenan, locust bean gum, agar, glucomannan, xanthan gum, tamarind gum, pectin, sodium alginate, gum arabic, or the like; celluloses such as crystalline cellulose, powdered cellulose, hydroxypropyl cellulose, methylcellulose, or the like; starches such as pregelatinized starch, starch paste, or the like; synthetic polymers such as polyvinylpyrrolidone, carboxyvinyl polymer, polyvinyl alcohol, or the like; and inorganic compounds such as calcium hydrogen phosphate, calcium carbonate, synthetic hydrotalcite, magnesium aluminosilicate, or the like; and the like. These binders may be used alone or in combination of two or more.

Examples of the fluidizing agent include those classified as fluidizing agents in the "Dictionary of Pharmaceutical Additives" (published by Yakuji Nipposha Co., Ltd.), such as silicon compounds such as hydrous silicon dioxide, light anhydrous silicic acid, or the like. These fluidizing agents may be used alone or in combination of two or more.

Examples of the lubricants include those classified as lubricants in the "Dictionary of Pharmaceutical Additives" (published by Yakuji Nipposha Co., Ltd.), such as magnesium stearate, calcium stearate, stearic acid, sucrose fatty acid esters, talc, or the like. These lubricants may be used alone or in combination of two or more.

Examples of the flavoring agents include those classified as flavoring agents in the "Dictionary of Pharmaceutical Additives" (published by Yakuji Nipposha Co., Ltd.), such as glutamic acid, fumaric acid, succinic acid, citric acid, sodium citrate, tartaric acid, malic acid, ascorbic acid, sodium chloride, 1-menthol, or the like. These flavoring agents may be used alone or in combination of two or more.

Examples of the flavoring agents include those classified as flavoring agents and fragrances in the "Dictionary of Pharmaceutical Additives" (published by Yakuji Nipposha Co., Ltd.), such as oils such as an orange, vanilla, strawberry, yogurt, menthol, fennel oil, cinnamon oil, spruce oil, and peppermint oil, or the like; green tea powder; and the like. These flavoring agents and fragrances may be used alone or in combination of two or more.

Examples of the coloring agents include those coloring agents classified as coloring agents in the "Dictionary of Pharmaceutical Additives" (published by Yakuji Nipposha Co., Ltd.), such as food dyes such as Food Red No. 3, Food Yellow No. 5, Food Blue No. 1, or the like, sodium copper chlorophyllin, titanium oxide, riboflavin, or the like. These coloring agents may be used alone or in combination of two or more.

Examples of the sweeteners include those classified as sweeteners in the "Dictionary of Pharmaceutical Additives" (published by Yakuji Nipposha Co., Ltd.), such as aspartame, saccharin, dipotassium glycyrrhizinate, stevia, maltose, maltitol, starch syrup, powdered Amacha, or the like. These sweeteners may be used alone or in combination of two or more.

### <Method for producing pharmaceutical composition according to the present embodiment>

The method for producing a pharmaceutical composition according to the present embodiment is a method for producing the pharmaceutical composition according to the present embodiment, in which the cellulose powder according to the present embodiment and a pharmaceutically active ingredient are used as raw materials.

In the method for producing a pharmaceutical composition according to the present embodiment, the raw material cellulose powder according to the present embodiment is preferably stored in a gas having a nitrogen dioxide concentration of 0.05 ppm or less and a nitrogen trioxide concentration of 0.05 ppm or less (e.g., an atmosphere in which the nitrogen dioxide concentration is controlled to 0.05 ppm or less and the nitrogen trioxide concentration is controlled 0.05 ppm or less) until the start of production. By storing the raw material in an environment in which the nitrogen dioxide and nitrogen trioxide concentrations are controlled to be low until the start of production, the amounts of nitrite nitrogen and nitrate nitrogen introduced from the raw material to the pharmaceutical composition can be kept low, thereby suppressing the generation of nitrosamines in the pharmaceutical composition. In the production of the pharmaceutical composition according to the present embodiment, raw materials other than the cellulose powder are also preferably stored in a gas having a nitrogen dioxide concentration of 0.05 ppm or less and a nitrogen trioxide concentration of 0.05 ppm or less until the start of production.

In the method for producing a pharmaceutical composition according to the present embodiment, it is preferable that all steps from the start to the end of production be carried out in a gas having a nitrogen dioxide concentration of 0.05 ppm or less and a nitrogen trioxide concentration of 0.05 ppm or less (for example, an atmosphere in which the nitrogen dioxide concentration is controlled to 0.05 ppm or less and the nitrogen trioxide concentration is controlled to 0.05 ppm or less). This makes it possible to keep the amounts of nitrite nitrogen and nitrate nitrogen introduced from the atmosphere during the production process low, thereby suppressing the generation of nitrosamines in the pharmaceutical composition.

In the method for producing a pharmaceutical composition according to the present embodiment, the cellulose powder according to the present embodiment is used as the raw cellulose powder to produce a pharmaceutical composition with low nitrosamine generation, and the composition can be produced by conventional methods. For example, when the pharmaceutical composition is in the form of a tablet, the composition can be produced by preparing a mixture containing cellulose powder and a pharmaceutically active ingredient and directly compressing the mixture (direct tableting method), or by granulating the mixture and then compressing it (granule compression method). Other methods that can be used include a post-pulverization method (a method in which the pharmaceutically active ingredient, cellulose powder, and optionally other additives are mixed, granulated to form granules, and then further mixed with cellulose powder and optionally other additives, and compression-molded by conventional methods), a method for producing a polynuclear tablet using a pre-compressed tablet as the core, and a method for producing a multi-layer tablet in which multiple pre-compressed compacts are stacked and re-compressed.

The method of tableting (compression molding) is not particularly limited as long as it is a commonly used method, and examples thereof include a method of compressing into a desired shape using a mortar and pestle, a method of compressing into a sheet in advance and then cutting into the desired shape, and the like. Examples of the compression molding machines include roller presses such as hydrostatic presses, briquetting roller presses, and smooth roller presses, single punch tablet presses, rotary tablet presses, and the like.

Examples of the granulation methods when granulation is performed include dry granulation, wet granulation, heat granulation, spray granulation, and microencapsulation. Specific examples of the effective wet granulation methods include fluidized bed granulation, stirring granulation, extrusion granulation, crushing granulation, and rolling granulation. Methods for drying the granulated material include hot air heating (shelf drying, vacuum drying, fluidized bed drying), conduction heat transfer (pan type, shelf box type, drum type), and freeze drying. In the hot air heating method, hot air is directly brought into contact with the material, and evaporated moisture is simultaneously removed.

### (Examples)

The present invention will be described based on the Examples, but the embodiments of the present invention are not limited to the descriptions of these Examples.

The methods for measuring and evaluating the various physical properties in the Examples and the Comparative Examples are as follows.

### <Measurement of content in cellulose powder>

### (1) Measurement of nitrite ions and nitrate ions

The nitrite ion and nitrate ion contents of the cellulose powder were measured by suppression ion chromatography under the following conditions. The nitrite ion and nitrate ion contents of the natural cellulosic material were also measured in the same manner.

Column: Anion exchange chromatography column (inner diameter 4.6 mm × 15 cm)
Mobile phase: Sodium carbonate/sodium bicarbonate eluent
Flow rate: 1.0 mL/min
Oven temperature: 40°C
Sample injection volume: 30 to 100 µL
Detector: UV-VIS detector (wavelength 210 nm)
LOD (detection limit): 0.008 µg/g

### (2) Measurement of sulfur

The sulfur content of the cellulose powder was measured by automatic combustion analysis using ion chromatography.

Specifically, approximately 50 mg of the sample was placed on a quartz board and burned in a combustion furnace at 1000°C, and the gasified components were bubbled into an absorption liquid. The resulting absorption liquid was analyzed using a CT device (model: INTEGRATION, manufactured by Thermo Fisher Scientific) to quantify sulfur. The detection limit was 0.1 ppm.

### (3) Measurement of hydrogen peroxide

The hydrogen peroxide content of the cellulose powder was measured by the oxygen electrode method.

Specifically, 2 g of sample was extracted with 0.2 mol/L phosphate buffer containing 0.5% potassium bromate, and then filtered under ice cooling to obtain a filtrate. 20 mL of the obtained filtrate was sampled and analyzed by the oxygen electrode method to quantify hydrogen peroxide. A hydrogen peroxide meter (SUPER ORITECTOR MODEL 5 (trade name), manufactured by Central Scientific Co., Ltd.) was used for the measurement, and 2 mL of the filtrate was injected into the measuring instrument for analysis. The detection limit was 0.1 ppm.

### (4) Measurement of iron

The iron content of the cellulose powder was measured by inductively coupled plasma mass spectrometry (ICP-MS).

The measurement samples were pretreated using a closed-system acid decomposition method. After collecting the sample and adding sulfuric acid, the sample was subjected to thermal decomposition treatment using a microwave sample decomposition device (product name: ETHOS UP, manufactured by Milestone Corporation). The sample was then filled up to a constant volume with ultrapure water and used as the analytical sample.

The prepared analytical sample was analyzed using an inductively coupled plasma mass spectrometer (model: iCAP RQ, manufactured by Thermo Fisher Scientific) to measure ⁵⁶Fe. The detection limit was 0.1 ppm.

### (5) Measurement of ammonia

The ammonia content of the cellulose powder was measured by ion chromatography in accordance with JIS K0127.

First, 3 g of sample (crystalline cellulose powder) was weighed into a 100 mL glass beaker, 60 mL of pure water was added, and the mixture was stirred at a constant stirring force for 20 minutes. After stirring, the mixture was filtered using quantitative filter paper (5C, manufactured by Advantec). The collected filtrate was used as the analytical sample.

The prepared analytical sample was analyzed using an ion chromatograph (model: INTEGRION, manufactured by Thermo Fisher Scientific) to measure ammonia. The ion chromatograph was equipped with a separation column (CS12 column), a guard column (CG12 column), and an electrical conductivity detector. The detection limit was 0.1 ppm.

### <Measurement of content in gas>

### (1) Measurement of nitrogen dioxide and nitrogen trioxide concentrations

The concentrations of nitrogen dioxide and nitrogen trioxide in the gas were measured by the same suppression ion chromatography method as used to measure the concentrations of nitrite ions and nitrate ions in the cellulose powder.

The atmospheric samples used for the measurements were collected for a predetermined period of time using a DSD-TEA sampler. After the sampler was collected, it was collected and extracted with pure water to prepare the analytical sample.

### <Measurement of content in water>

### (1) Measurement of nitrite nitrogen and nitrate nitrogen

The content of nitrite nitrogen in the water and the total content of nitrite nitrogen and nitrate nitrogen in the water were measured by the same suppression ion chromatography method as used to measure the nitrite ion and nitrate ion in the cellulose powder.

### (Example 1)

Six types of cellulose powder were used to prepare tablets containing active ingredients that may generate nitrosamines, and the amount of nitrosamine generated was compared.

### (1) Preparation of cellulose powder

Seven types of commercially available SP pulp (all with nitrite ion concentrations below 0.008 ppm (detection limit) and nitrate ion concentrations of 0.8 to 4.6 ppm) were appropriately blended as raw materials to prepare six types of cellulose powders (A to F) with different raw material pulps. The nitrite ion concentrations, nitrate ion concentrations, hydrogen peroxide concentrations, sulfur concentrations, iron concentrations, and ammonia concentrations of the raw material pulps (A to F) used were measured. The raw material pulp was processed into a cotton-like form, and then the concentration of each component was measured using the same measurement method as for the cellulose powder. The measurement results are shown in Table 1. Note that when the concentration was below the detection limit, the detection limit was used as the concentration of the sample.

**[Table 1]**

| Raw material pulp | NO₂⁻ [ppm] | NO₃⁻ [ppm] | H₂O₂ [ppm] | S [ppm] | Fe [ppm] | NH₃ [ppm] |
|---|---|---|---|---|---|---|
| A | 0.008 | 0.27 | 0.1 | 15.3 | 0.75 | 0.3 |
| B | 0.008 | 0.68 | 0.1 | 18.2 | 0.62 | 0.5 |
| C | 0.008 | 0.35 | 0.2 | 19.9 | 0.54 | 0.8 |
| D | 0.008 | 0.45 | 0.1 | 19.2 | 0.73 | 0.4 |
| E | 0.008 | 0.38 | 0.1 | 16.4 | 0.55 | 0.4 |
| F | 0.008 | 0.49 | 0.1 | 16.8 | 0.62 | 0.5 |

Powdered cellulose (A to F) was prepared from each raw pulp (A to F). From the start to the end of the preparation of cellulose powder from the raw pulp, the process was carried out in an atmospheric environment with a nitrogen dioxide concentration of 0.03 ppm or less and a nitrogen trioxide concentration of 0.03 ppm or less. Furthermore, the water (pure water) used in the preparation process had a nitrite nitrogen concentration of 0.004 ppm or less and a total of nitrite nitrogen and nitrate nitrogen of 0.1 ppm or less.

Specifically, 2 kg of raw pulp was shredded and placed in 30 L of a 0.05% aqueous hydrochloric acid solution and hydrolyzed for 70 minutes at 145°C while stirring (stirring speed: 234 rpm) with a low-speed stirrer (Ikebukuro Horo Kogyo Co., Ltd., 30 LGL reactor, impeller diameter: approximately 30 cm). The resulting acid-insoluble residue was filtered using a Nutsche filter, and the filtration residue (insoluble residue) was washed four times with 70 L of pure water and neutralized with ammonia water. The mixture was then placed in a 90 L plastic bucket, pure water was added, and a cellulose dispersion was prepared while stirring (stirring speed: 500 rpm) with a Three-One Motor (HEIDON, Type BLh1200, 8M/M, impeller diameter: approximately 10 cm). The cellulose dispersion was spray-dried (liquid supply rate 6 L/h, inlet temperature 180-220°C, outlet temperature 50-70°C) to obtain cellulose powders A to C, E, and F.

In addition, 2 kg of raw pulp was shredded and placed in 30 L of 0.08% aqueous hydrochloric acid solution. The mixture was stirred (stirring speed 234 rpm) with a low-speed mixer (Ikebukuro Horo Kogyo Co., Ltd., 30 LGL reactor, blade diameter approximately 30 cm) and hydrolyzed at 135°C for 80 minutes. The resulting acid-insoluble residue was filtered using a Nutsche filter, and the filtration residue (insoluble residue) was washed four times with 70 L of pure water. After neutralization with ammonia water, the mixture was placed in a 90 L plastic bucket, pure water was added, and a cellulose dispersion was prepared by stirring (stirring speed 500 rpm) with a Three-One Motor (HEIDON, Type BLh1200, 8M/M, blade diameter approximately 10 cm). The cellulose dispersion was spray-dried (liquid supply rate 6 L/h, inlet temperature 180-220°C, outlet temperature 50-70°C) to obtain cellulose powder D.

The weight-average particle diameter (µm), apparent specific volume (cm³/g), apparent tapping density (g/cm³), angle of repose (°), nitrite ion concentration, nitrate ion concentration, hydrogen peroxide concentration, sulfur concentration, iron concentration, and ammonia concentration were measured for the prepared cellulose powders A to F and the commercially available cellulose powders G to J. The results are shown in Tables 2 and 3. When the concentration was below the detection limit, the detection limit was used as the concentration of the sample. In the tables, underlined values indicate values below the detection limit.

**[Table 2]**

| Cellulose powder | Weight-average particle diameter [µm] | Apparent specific volume [cm³/g] | Apparent tapping density [g/cm³] | Angle of repose [°] |
|---|---|---|---|---|
| A | 94 | 3.44 | 0.396 | 40 |
| B | 96 | 3.27 | 0.401 | 39 |
| C | 99 | 3.21 | 0.410 | 38 |
| D | 52 | 4.47 | 0.361 | 47 |
| E | 98 | 3.23 | 0.431 | 41 |
| F | 103 | 3.26 | 0.414 | 40 |
| G | 98 | 3.23 | 0.408 | 38 |
| H | 99 | 3.33 | 0.408 | 41 |
| I | 109 | 3.52 | 0.429 | 42 |
| J | 104 | 3.22 | 0.416 | 41 |

**[Table 3]**

| Cellulose powder | NO₂⁻ [ppm] | NO₃⁻ [ppm] | H₂O₂ [ppm] | S [ppm] | Fe [ppm] | NH₃ [ppm] |
|---|---|---|---|---|---|---|
| A | 0.008 | 0.190 | 0.2 | 14.1 | 0.54 | 2.2 |
| B | 0.012 | 0.036 | 0.2 | 15.3 | 0.44 | 1.5 |
| C | 0.012 | 0.038 | 0.2 | 18.1 | 0.34 | 1.6 |
| D | 0.008 | 0.082 | 0.1 | 24.7 | 0.52 | 1.5 |
| E | 0.008 | 0.064 | 0.2 | 17.2 | 0.51 | 2.1 |
| F | 0.008 | 0.110 | 0.2 | 18.9 | 0.47 | 1.8 |
| G | 0.019 | 2.400 | 0.3 | 22.3 | 0.34 | 1.2 |
| H | 0.340 | 8.300 | 0.7 | 35.2 | 0.71 | 1.8 |
| I | 0.094 | 8.600 | 0.6 | 30.8 | 0.68 | 1.7 |
| J | 0.190 | 8.100 | 0.8 | 31.3 | 0.64 | 1.6 |

### (2) After preparation and storage of tablets

Cellulose powders A to J and tablets were prepared using MCPA, which has a dimethylaminomethyl group in its skeleton and has been reported to pose a risk of generating nitrosamines, as the active ingredient.

Specifically, 300 mg tablets were prepared by direct compression from a mixture of cellulose powder and MCPA in a mass ratio of 5:5, and then stored for one month in an atmospheric environment at 50°C, 80% relative humidity, and with a nitrogen dioxide concentration of 0.03 ppm or less and a nitrogen trioxide concentration of 0.03 ppm or less.

### (3) Measurement of nitrosamine content

The content of nitrosamines (NDMA, NDEA) in the tablets after storage was measured by gas chromatography-mass spectrometry (GC-MS/MS).

First, the tablet was pulverized in a mortar, and approximately 25 mg of the pulverized material was precisely weighed. 4 mL of 1 mol/L NaOH solution was added to the pulverized material, followed by ultrasonic irradiation for 10 minutes. Then, exactly 4 mL of internal standard (NDMA-d₆, NDEA-d₁₀) solution was added, and the mixture was subjected to permeation treatment for 15 minutes, followed by centrifugation (3000 rpm × 5 minutes). After centrifugation, the lower layer was collected, and the solution filtered was used as the sample solution.

The prepared sample solution was analyzed using a GC-MS/MS system (Shimadzu Corporation) to quantitatively measure the nitrosamines in the sample solution. The GC-MS/MS system used was equipped with a GCMS gas chromatograph (model number: CG-2030), a GCMS mass spectrometer (model number: GCMS-TQ8050NS), and a GCMS autoinjector (model number: AOC-6000plus) (all manufactured by Shimadzu Corporation). The detection limit was 0.05 µg/g.

**[Table 4]**

| Cellulose powder | NDMA [µg/g] | NDEA [µg/g] |
|---|---|---|
| A | 0.030 | ND |
| B | 0.026 | ND |

| | | |
|---|---|---|
| C | 0.065 | ND |
| D | 0.056 | ND |
| E | 0.048 | ND |
| F | 0.034 | ND |
| G | 0.202 | ND |
| H | 0.684 | ND |
| I | 0.587 | ND |
| J | 0.884 | ND |

The measurement results for tablets prepared using each cellulose powder are shown in Table 4. In the table, "ND" indicates that the substance was not prepared.

According to Table 2, the weight-average particle diameter (µm), apparent specific volume (cm³/g), apparent tapping density (g/cm³), and angle of repose (°) of the nine types of cellulose powders other than cellulose powder D were all similar, indicating that the physical properties of these cellulose powders were similar. On the other hand, according to Tables 3 and 4, the tablets made using cellulose powders G to J, which had significantly high nitrite ion and nitrate ion concentrations, also had clearly high NDMA concentrations. These results demonstrate that differences in powder physical properties have little effect on the amount of nitrosamine generated. Furthermore, cellulose powder D had a higher apparent specific volume and apparent tapping density than the other cellulose powders, but generated a higher amount of nitrosamine than cellulose powders A or B. From this result, it was also considered that differences in powder physical properties did not affect the amount of nitrosamine generated.

Comparing the tablets obtained from cellulose powders A, D, and F, the NDMA concentration in the tablet made from cellulose powder D, which had the lowest total concentration of nitrite ions and nitrate ions, was higher than the NDMA concentration in the tablets made from cellulose powders A or F, which had higher total concentrations of nitrite ions and nitrate ions. These results confirmed that the amount of nitrosamines generated in the tablets is affected by factors other than the nitrate ion concentration derived from the cellulose powder.

As shown in Tables 3 and 4, the higher the hydrogen peroxide and sulfur concentrations of the cellulose powder, the higher the NDMA concentration. Similarly, the higher the iron, nitrate, and nitrite concentrations of the cellulose powder, the higher the NDMA concentration. These results indicate that hydrogen peroxide, sulfur, iron, nitrite, and nitrate ions derived from the cellulose powder affect the generation of nitrosamines. On the other hand, no significant correlation was observed between the ammonia concentration and NDMA concentration of the cellulose powder.

### (Example 2)

The effect of the nitrite ion concentration in the gas used for spray-drying on the resulting cellulose powder and the tablets prepared therefrom was investigated.

First, raw material pulp A was hydrolyzed in the same manner as cellulose powder A used in Example 1, and the acid-insoluble residue was washed and neutralized, and then pure water was added and stirred to prepare a cellulose dispersion. The obtained cellulose dispersion was spray-dried with gases having different nitrite ion concentrations, and the nitrite ion concentrations and nitrate ion concentrations of the obtained cellulose powder were measured.

### (1) Preparation in an atmospheric environment with a sufficiently low concentration of nitrogen dioxide

The cellulose dispersion was spray-dried using a spray dryer in an environment where the atmospheric nitrogen dioxide concentration was 0.020 ppm. As a result of analyzing the resulting cellulose powder, the nitrite ion concentration was 0.011 ppm and the nitrate ion concentration was 0.072 ppm. These results demonstrate that when the nitrogen dioxide concentration of the gas used for spray-drying is sufficiently low, a cellulose powder with sufficiently low concentrations of both nitrite ion and nitrate ion can be obtained.

### (2) Preparation in an atmospheric environment with a high concentration of nitrogen dioxide (A)

A cellulose dispersion was spray-dried using a spray dryer in an environment where the atmospheric nitrogen dioxide concentration was greater than 0.020 ppm. Spray-drying was performed with and without a chemical filter installed at the spray-drying gas inlet of the spray dryer, which can efficiently remove acidic gases such as nitrogen dioxide. The nitrite ion and nitrate ion concentrations of the spray-drying gas and the resulting cellulose powder were measured. Filter A ("RM2B90", manufactured by Osaka Gas Chemicals Co., Ltd.) was used as the chemical filter. The results are shown in Table 5.

**[Table 5]**

| | Gas for spray-drying | | Cellulose powder | |
|---|---|---|---|---|
| Filter | NO₂⁻ [ppm] | NO₃⁻ [ppm] | NO₂⁻ [ppm] | NO₃⁻ [ppm] |
| No | 0.130 | 0.130 | 0.043 | 0.340 |
| Yes | 0.020 | 0.020 | 0.008 | 0.095 |

As shown in Table 5, the chemical filter reduced both the nitrite ion concentration and the nitrate ion concentration in the spray-drying gas to 0.020 ppm or less. Furthermore, the cellulose powder obtained by spray-drying with a filter had significantly lower concentrations of both nitrite ion and nitrate ion than the powder obtained by spray-drying without a filter. These results demonstrate that a lower nitrogen dioxide concentration in the gas used for spray-drying can reduce the nitrite ion and nitrate ion concentrations in the resulting cellulose powder, and that the use of a chemical filter can control the nitrogen dioxide concentration in the spray-drying gas even in an atmosphere with a high nitrogen dioxide concentration.

### (3) Preparation in an atmospheric environment with a high concentration of nitrogen dioxide (B)

A cellulose dispersion was spray-dried using a spray dryer in an environment where the atmospheric nitrogen dioxide concentration was greater than 0.020 ppm. Spray-drying was performed in the same manner as in (2) described above, except that filter B ("Philofresh VZG" manufactured by Japan Vilene Co., Ltd.) was used as the chemical filter. The nitrite ion concentration and nitrate ion concentration of the spray-drying gas and the resulting cellulose powder were measured. The results are shown in Table 6.

**[Table 6]**

| | Gas for spray-drying | | Cellulose powder | |
|---|---|---|---|---|
| Filter | NO₂⁻ [ppm] | NO₃⁻ [ppm] | NO₂⁻ [ppm] | NO₃⁻ [ppm] |
| No | 0.130 | 0.028 | 0.043 | 0.270 |
| Yes | 0.020 | 0.010 | 0.008 | 0.100 |

As shown in Table 6, when filter B was used, as when filter A was used, both the nitrite ion concentration and the nitrate ion concentration in the spray-drying gas could be reduced to 0.020 ppm or less, and the cellulose powder obtained by spray-drying with a filter had significantly lower concentrations of both nitrite ion and nitrate ion than that obtained by spray-drying without a filter.

### INDUSTRIAL APPLICABILITY

The cellulose powder according to the present embodiment has a reduced content of components that affect the generation of nitrosamines, making it extremely useful as a raw material for pharmaceuticals that require high safety.

## Claims

1. A cellulose powder, which has a nitrate ion content of 1.00 ppm or less and a sulfur content of 30.0 ppm or less.

2. The cellulose powder according to claim 1, which has a hydrogen peroxide content of 0.40 ppm or less.

3. A cellulose powder, which has a nitrate ion content of 1.00 ppm or less and a hydrogen peroxide content of 0.40 ppm or less.

4. The cellulose powder according to claim 1, wherein the nitrite ion content is 0.200 ppm or less.

5. The cellulose powder according to claim 1, wherein an iron content is 0.10 ppm or more and 0.80 ppm or less.

6. A pharmaceutical composition comprising the cellulose powder according to any one of claims 1 to 5 and a pharmaceutically active ingredient.

7. The pharmaceutical composition according to claim 6, wherein the pharmaceutically active ingredient is a secondary amine, a tertiary amine, or a quaternary amine.

8. The pharmaceutical composition according to claim 6, which is a tablet.

9. A method for producing a cellulose powder, comprising
hydrolyzing a natural cellulosic material, washing an insoluble residue in a cellulose dispersion after hydrolysis, and then spray-drying the washed cellulose, wherein
(1) the natural cellulosic material has a nitrite ion content of 0.010 ppm or less or a sulfur ion content of 30.0 ppm or less, and a nitrate ion content of 5.0 ppm or less; or
(3) the spray-drying is carried out in a gas having a nitrogen dioxide concentration of 0.05 ppm or less or a nitrogen trioxide concentration of 0.05 ppm or less.

10. The method for producing a cellulose powder according to claim 9, comprising
hydrolyzing a natural cellulosic material, washing an insoluble residue in a cellulose dispersion after hydrolysis, and then spray-drying the washed cellulose, wherein
(1) the natural cellulosic material has a nitrite ion content of 0.010 ppm or less or a sulfur ion content of 30.0 ppm or less, and a nitrate ion content of 5.0 ppm or less; and
(3) the spray-drying is carried out in a gas having a nitrogen dioxide concentration of 0.05 ppm or less.

11. The method for producing a cellulose powder according to claim 9 or 10, wherein
(2) water used in one or more processes selected from the group consisting of the hydrolysis reaction of the natural cellulosic material, the washing of the insoluble residue, and the spray-drying has a nitrite nitrogen content of 0.02 ppm or less, or a total content of nitrite nitrogen and nitrate nitrogen of 5.0 ppm or less.

12. A method for producing a pharmaceutical composition, comprising using the cellulose powder according to any one of claims 1 to 5 and a pharmaceutically active ingredient as raw materials.

13. The method for producing a pharmaceutical composition according to claim 12, wherein the cellulose powder is stored in a gas having a nitrogen dioxide concentration of 0.05 ppm or less and a nitrogen trioxide concentration of 0.05 ppm or less until a start of production.

14. The method for producing a pharmaceutical composition according to claim 12, wherein the pharmaceutical composition is produced in a gas having a nitrogen dioxide concentration of 0.05 ppm or less and a nitrogen trioxide concentration of 0.05 ppm or less.

15. The method for producing a pharmaceutical composition according to claim 12, wherein the pharmaceutically active ingredient is a secondary amine, a tertiary amine, or a quaternary amine.

16. The method for producing a pharmaceutical composition according to claim 12, wherein the pharmaceutically active ingredient is a sartan compound, a compound having a dimethylaminomethyl group, or a biguanide compound.

17. The method for producing a pharmaceutical composition according to claim 12, wherein a mixture containing the cellulose powder and the pharmaceutically active ingredient is directly compressed into tablets, or granulated and then compressed into tablets.
